# EUROPEAN PATENT APPLICATION

(11) **EP 3 685 775 A1**
(43) Date of publication of application: **29.07.2020**
(21) Application number: 19305102.6
(22) Date of filing: 25.01.2019
(51) Int. Cl.: A61B 17/34

(54) **TROCAR FIXING DEVICE**

(71) Applicant: CHU de NICE, 06200 Nice (FR)
(72) Inventor: SEJOR, Eric, 06000 Nice (FR)
(74) Representative: Osha Liang

(57) **Abstract**

The disclosure relates to a device (1) for fixing a trocar (2) at a predetermined position, the device (1) comprising: an open annular support (70) comprising two free ends (70a, 70b) defining a gap (70c) therebetween, a first dome (40) rotatably supported by the open annular support (70) and comprising a first meridian slot opening (41) ending in the gap (70c), and a second dome (50) rotatably supported by the open annular support (70) and comprising a second meridian slot opening (51) ending in the gap (70c), the first dome (40) and the second dome (50) being juxtaposed to each other so that the second dome (50) is rotatable with the first dome (40) and the first meridian slot opening (41) matches the second meridian slot opening (51) to define a common meridian slot opening (80) for receiving a trocar (2) freely movable therethrough, the open annular support (70) further comprising fixing means (22, 32) for fixing the first dome (40) and the second dome (50) at a predetermined angular position and for fixing the trocar (2) received in the common meridian slot opening (80) at a predetermined meridian position.

## Description

### Field of the disclosure

The present disclosure relates to devices and methods for fixing a trocar at a predetermined position. The trocar may be fixed in an operational access, for example for positioning a medical instrument percutaneously inserted into a tissue of a patient.

### Technical background

Surgery requires increased access to internal tissues through minimally invasive surgical procedures. The term "minimally invasive" refers to a wide range of surgical procedures including laparoscopic, thoracoscopic, arthroscopic, endoscopic, and natural orifice transluminal procedures.

During certain minimally invasive procedures, trocars percutaneously inserted into a patient's body may be used to provide a port for surgical instruments.

Development in minimally invasive surgery has resulted in increasingly complex procedures requiring the use of numerous surgical instruments, as well as precise manipulations within the body. The weight of surgical instruments and even trocars often requires additional surgeons in order to uphold the position of instruments and/or trocars.

Accordingly, there is a need for devices and methods that allow to maintain a percutaneous surgical instrument at a desired position and/or orientation, thereby freeing the users for other tasks or even giving the opportunity for one to operate alone.

Among the prior art devices, a trocar fixation device is described in patent document US 9,629,659. This trocar fixation device includes a retaining ring, a ball joint disposed within and movable relative to the retaining ring, and a locking mechanism. The ball joint defines a channel configured to receive an instrument therethrough.

However, such a device may require long trocars, since the described height of the trocar fixation device limits the room for movement of the trocar in the device.

### Summary of the disclosure

In what follows, the term "comprise" is synonym of (means the same as) "include" and "contains", is inclusive and open and does not exclude other non-recited elements.

According to a first aspect, the disclosure relates to a device for fixing a trocar at a predetermined position, the device comprising an open annular support comprising two free ends defining a gap therebetween, a first dome rotatably supported by the open annular support and comprising a first meridian slot opening ending in the gap, and a second dome rotatably supported by the open annular support and comprising a second meridian slot opening ending in the gap. The first dome and the second dome are juxtaposed to each other so that the second dome is rotatable with the first dome and the first meridian slot opening matches the second meridian slot opening to define a common meridian slot opening for receiving a trocar freely movable therethrough. The open annular support further comprises a fixing means for fixing the first dome and the second dome at a predetermined angular position and for fixing the trocar received in the common meridian slot opening at a predetermined meridian position.

Accordingly, a trocar may be inserted in the common meridian slot through the gap of the open annular support. In this manner, the trocar may be percutaneously inserted into the patient's body prior to being inserted in the device.

The predetermined angular position depends on the degree of rotation of the first and second dome. Since, according to one or more embodiments, each of the first dome and second dome may rotate by 360°, according to one or more embodiments, the predetermined angular position may be any angular position from 0° to 360°.

The predetermined meridian position may be any position along the common meridian slot opening, the only restriction of movement in this direction being due to the vertical dimension of the open annular support.

This configuration results in an increased room for movement of the trocar in different directions in the device, at least both in terms of angular position which may be reached by the trocar on the plane of the open annular support and in terms of meridian position which may be reached by the trocar along the common meridian slot opening.

According to one or more embodiments, the trocar may pivot along the common meridian slot opening, for example over a predetermined angle of rotation, which may be for example of more than 100°, such as for example from 100° to 160°. The predetermined meridian position depends on the radius of curvature of the first and second domes, which, according to one or more embodiments, may range from 20 mm to 50 mm.

According to one or more embodiments, the trocar may pivot around an axis perpendicular to the longitudinal axis of the open annular support.

The device according to the present disclosure allows, in a simple way, to fix a trocar at a predetermined position, such as in an operational access, which may be a position corresponding to any angular position on a plane of the open annular support and any meridian position along the common meridian slot opening, such as for example up to 160° and even higher, because the longitudinal dimension or height of the open annular support is restricted with respect to the longitudinal dimension or height of the supports of prior art devices.

For example, the trocar may be fixed for positioning a medical instrument percutaneously inserted into a patient's body, for example into a tissue of a patient. The trocar may be also percutaneously inserted into a patient's body. The device may be used, for example, during a surgical intervention.

The device according to the present disclosure may then afford, among others, the following advantages:
- the liberation of hands: the trocar, once correctly positioned within a body cavity of a patient by a surgeon, may be fixed at a predetermined position. Thus, the trocar does not have to be physically held by a member of a surgical team throughout the whole duration of a surgical intervention. Less human assistance is then needed, which facilitates and lowers the costs of the intervention.
- the surgeon's access to the patient's body may be less hindered. As less operators may be needed to hold the medical instrument in place, more available space may be cleared.

According to one or more embodiments, the device further comprises a meridian fixing means for receiving and holding the trocar in the common meridian slot opening along a predetermined axis, the meridian fixing means being movable along the common meridian slot opening.

According to one or more embodiments, the meridian fixing means comprises a first portion and a second portion, the first portion being configured to receive and hold the trocar in the common meridian slot opening along the predetermined axis, and the second portion being disposed between the first dome and the second dome and being configured to be fixed by the fixing means at the predetermined meridian position.

According to one or more embodiments, the second portion comprises a wall having a radius of curvature in accordance with the curvature of the first dome and the second dome.

According to one or more embodiments, the second portion comprises a curved wall including two curved wings extending on opposite sides with respect to the first portion and intended to be disposed on the first dome.

According to one or more embodiments, the predetermined axis along which the trocar may be received and held in the common meridian slot opening by the first portion of the meridian fixing means may be any axis at an angle of from -30° to +30° with respect to the axis perpendicular to the plane of the second portion of the meridian fixing means.

According to one or more embodiments, the first portion comprises a housing configured to receive and hold a shaft of the trocar along the predetermined axis. According to one or more embodiments, the first portion comprises a clip, for example a U-clip.

According to one or more embodiments, the second portion comprises at least one notch and each of the first dome and second dome comprises at least one groove for receiving the at least one notch in a movable manner therethrough, the at least one groove of each of the first dome and the second dome extending parallel to the respective meridian slot opening of the first dome and the second dome. Any configuration may be envisaged provided that the meridian fixing means remains movable along the common meridian slot opening.

According to one or more embodiments, the open annular support comprises: a first open annular element comprising two free ends defining a first annular element gap, and a second open annular element comprising two free ends defining a second annular element gap matching the first annular element gap. The fixing means comprises a first fixing means arranged on the first open annular element and a second fixing means arranged on the second open annular element and configured to cooperate with the first fixing means to engage the first open annular element with the second open annular element.

According to one or more embodiments, the first fixing means and the second fixing means comprise a notch and a protrusion, respectively, or viceversa.

According to one or more embodiments, the first fixing means and the second fixing means comprise a tapping and a threading, respectively, or viceversa.

According to one or more embodiments, the first dome is provided with a first open annular edge rotatably supported by the open annular support and comprising two free ends defining a first annular edge gap therebetween and the second dome is provided with a second open annular edge rotatably supported by the open annular support and comprising two free ends defining a second annular edge gap therebetween, the first annular edge gap matching the second annular edge gap.

According to one or more embodiments, the first dome further comprises at least one bumper arranged along the common meridian slot at the proximity of at least one of the two free ends of the first dome.

According to one or more embodiments, the second dome comprises at least one notch arranged along the common meridian slot at the proximity of at least one of the free ends of the second dome, the at least one notch receiving the at least one bumper.

Advantageously, said at least one bumper restricts the mobility of the meridian fixing means in the common meridian slot opening while ensuring the maximum range of movement of the trocar along the common meridian slot opening.

According to a second aspect, the disclosure relates to a method for fixing a trocar at a predetermined position, the method comprising the steps of: providing a device for fixing a trocar at a predetermined position according to the first aspect, inserting the trocar in the common meridian slot of the first dome and the second dome through the gap of the open annular support along a predetermined axis, moving the trocar inserted in the common meridian slot through the common meridian slot to a predetermined meridian position, rotating trocar to a predetermined angular position, and fixing the trocar at the predetermined meridian position and at the predetermined angular position by means of the fixing means.

According to one or more embodiments, the method further comprises inserting the trocar in the first portion of the meridian fixing means.

According to one or more embodiments, when the open annular support of the device according to the first aspect comprises first and second open annular elements, fixing the trocar comprises engaging the first open annular element with the second open annular element.

The embodiments described above are not exhaustive. In particular, it is understood that additional embodiments can be considered on the basis of different combinations of the explicitly described embodiments.

### Brief description of the drawings

The present disclosure will be better understood and other advantages and embodiments will become clear on reading the description that follows, given purely by way of indication and in no way limiting, and by referring to the appended figures
in which:
- FIG. 1 is a perspective view of a device according to one ore more embodiments of the present disclosure.
- FIG. 2 is a perspective view of a first open annular element of the device of FIGS. 1A and 1B.
- FIG. 3 is a perspective view of a second open annular element of the device FIGS. 1A and 1B.
- FIG 4. is a perspective view of a first dome of the device of FIGS. 1A and 1B.
- FIG. 5 is a perspective view of a second dome of the device of FIGS. 1A and 1B.
- FIG 6 is a perspective view of a meridian fixing means of the device of FIGS. 1A and 1B.
- FIGS. 7A and 7B are a side view and a perspective view, respectively, of the device of FIGS 1A and 1B.

### Detailed description of embodiments

Embodiments of the present disclosure will now be described in detail with reference to the accompanying figures. In the following detailed description of embodiments of the present disclosure, numerous specific details are set forth in order to provide a more thorough understanding of the present disclosure. However, it will be apparent to one of ordinary skill in the art that the present disclosure may be practiced without these specific details. In other instances, well-known features have not been described in detail to avoid unnecessarily complicating the description.

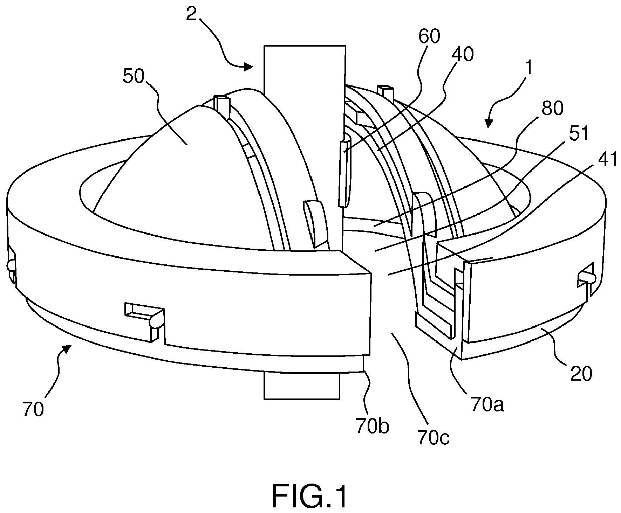

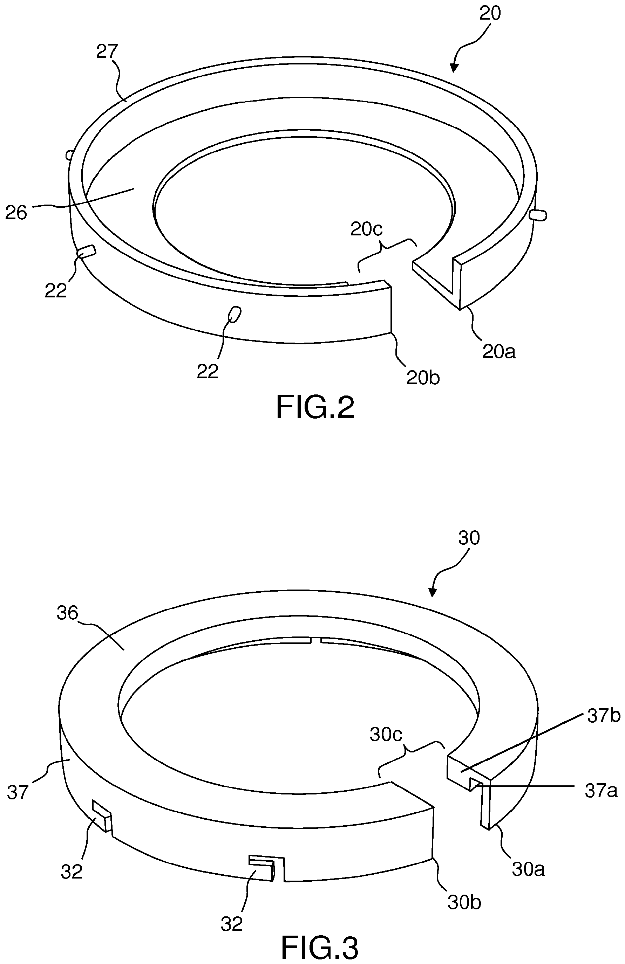

The following description provides a particular, non-limiting example of a device for fixing a trocar at a predetermined position according to one or more embodiments of the present disclosure. Said example of device and of components thereof is shown in FIG. 1 and in the following figures FIGs. 2-7. In these figures, the device is designated by reference number 1 and the trocar, when depicted, comprises a hollow cylindrical shaft, and is designated by reference number 2.

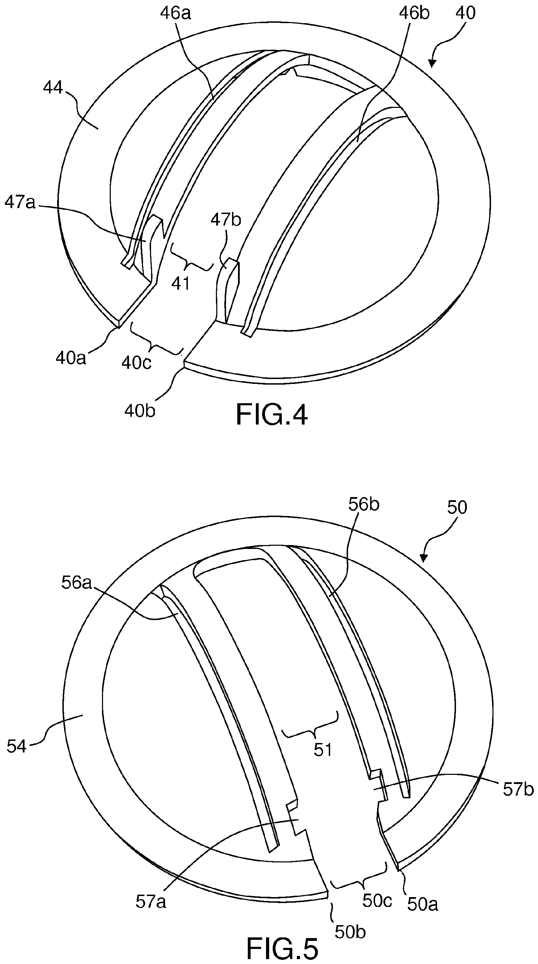

As shown in FIG.1, the device 1 comprises an open annular support 70 comprising two free ends 70a, 70b defining a gap 70c therebetween, a first dome 40 and a second dome 50, both rotatably supported by the open annular support 70. The first dome 40 and the second dome 50, which may have a radius of curvature for example ranging from 20 mm to 50 mm, comprise a first meridian slot opening 41 and a second meridian slot opening 51, respectively, each ending in the gap 70c, as also shown in FIGs. 4 and 5.

The first dome 40 and the second dome 50 are juxtaposed to each other so that the second dome 50 is rotatable with the first dome 40 and the first meridian slot opening 41 matches the second meridian slot opening 51 to define a common meridian slot opening 80 for receiving a trocar 2 freely movable therethrough.

As shown in FIGs. 4 and 5, each of the first dome 40 and second dome 50 comprises two grooves 46a, 46b, 56a, 56b extending parallel to and on opposite sides of the respective meridian slot opening 41, 51 of the first dome 40 and the second dome 50.

FIGs. 4 and 5 illustrate that the first dome 40 of device 1 is provided with a first open annular edge 44 rotatably supported by the open annular support 70 and comprising two free ends 40a; 40b defining a first annular edge gap 40c therebetween and the second dome 50 is provided with a second open annular edge 54 rotatably supported by the open annular support 70 and comprising two free ends 50a; 50b defining a second annular edge gap 50c therebetween. As shown in FIG. 1, the first open annular edge 44 and the second open annular edge 54 may be superimposed. Such superimposition may be provided by the open annular support 70 of device 1, which, as shown in FIG. 2, comprises an internal annular edge 26 suitable for receiving the first open annular edge 44 of the first dome 40, on which the second open annular edge 54 is superimposed, as shown in FIG. 1.

As further shown in FIG.1, the first annular edge gap 40c matches the second annular edge gap 50c. More generally, all previously mentioned gaps match each other, and meridian slot openings 41, 51 match each other, so as to allow the insertion of the trocar 2 through the gaps in the common meridian slot opening 80, as shown in FIG. 1.

According to the embodiment of FIG.1, the open annular support 70 comprises a first open annular element 20 and a second open annular element 30, which are also illustrated in FIGs. 2 and 3, respectively, and which are discussed in more detail in the following. However, other configurations of the open annular support 70 may be envisaged. For example, according to one or more embodiments of the present disclosure, the open annular support 70 may comprise an integrally formed open annular element capable of rotatably supporting the first dome 40 and the second dome 50.

In line with the device according to the present disclosure, the open annular support 70 in the example of device 1 depicted in FIGs. 1-3 and 7 further comprises a fixing means, indicated by reference numbers 22 and 32, for fixing the first dome 40 and the second dome 50 at a predetermined angular position and for fixing the trocar 2 received in the common meridian slot opening 80 at a predetermined meridian position.

As shown in FIGs. 2 and 3, the first open annular element 20 comprises two free ends 20a, 20b defining a first annular element gap 20c, and the second open annular element 30 comprises two free ends 30a, 30b defining a second annular element gap 30c matching the first annular element gap 20c.

In this embodiment, the fixing means 22, 32 comprises a first fixing means 22 which is arranged on the first open annular element 20 and a second fixing means 32 which is arranged on the second open annular element 30 and configured to cooperate with the first fixing means 32 to engage the first open annular element 20 with the second open annular element 30.

In the example of the device 1 depicted in the figures, the first fixing means 22 and the second fixing means 32 comprise a plurality of ball-shape protrusions and L-shape notches, respectively. According to one or more embodiments, however, a single couple notch/protrusion may be envisaged and different shapes of the couple notch/protrusion may be used. Moreover, according to one or more embodiments, the first fixing means 22 and the second fixing means 32 may comprise tapping(s) and threading(s), respectively, or viceversa, or any other cooperating means ensuring a reversible engagement of the first open annular element 20 with the second open annular element 30. As shown for example in FIG. 1, such cooperativity can between the first fixing means 22 and the second fixing means can be provided by a plurality of protrusion/notch couples, placed at predetermined intervals on the external edge of the open annular support. According to one or more embodiments, the number of used protrusion/notch couples may be comprised between 1 and 10, for example between 4 and 6.

As shown in FIG. 3, the second open annular element 30 further comprises an open annular groove 37a and an open annular protuberance 37b. The open annular groove 37a acts as a housing for an open annular wall 27 of the first open annular element 30. This configuration may help providing, among other advantages, a fluid rotation of the rotatably supported domes 40, 50.

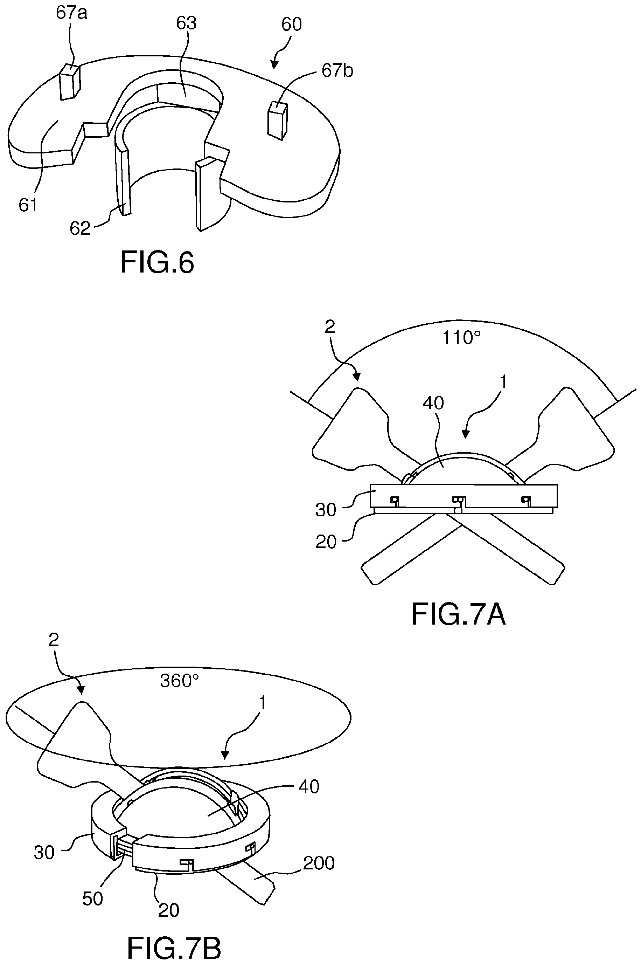

In this example, as shown in FIGs. 1 and 6, the device 1 further comprises a meridian fixing means 60 for receiving and holding the trocar 2 in the common meridian slot opening 80 along a predetermined axis, the meridian fixing means 60 being movable along the common meridian slot opening 80. As shown in FIG. 1, the meridian fixing means 60 may be disposed between the first dome 40 and the second dome 50. As shown in FIG. 6, said meridian fixing means 60 comprises a first portion 62 and a second portion 61.

The first portion 62 is configured to receive and hold the trocar 2 in the common meridian slot opening 80 along the predetermined axis. In this manner, by suitably configuring the first portion, not only the trocar may be firmly supported in the device, but the arrangement of the longitudinal axis of the trocar may be also selected, for example as a function of the type of surgery.

The second portion 61 is disposed between the first dome 40 and the second dome 50 in a movable manner along the common meridian slot opening 80 and is configured to be fixed by the fixing means 22, 32 at the predetermined meridian position, which, as discussed in more detail in the following, results in the possibility of positioning and fixing the trocar in an easy, quick and reliable manner.

In accordance with one or more embodiments, the second portion 61 comprises a wall having substantially the same radius of curvature of the first dome 40 and the second dome 50. With reference to the embodiments wherein the device further comprises a meridian fixing means, the second portion 61 depicted in FIGS. 1 and 6 comprises a curved wall including two curved wings extending on opposite sides with respect to the first portion 62. Both wings have a curved shape, in accordance with the curvature of the first and second domes 40, 50. This configuration may allow, among other advantages, to facilitate the movement of the second portion 61 along the common meridional slot opening 41, 51, before the fixing means fixes the first dome 40 and the second dome 50 (and thereby the trocar 2) at the predetermined angular position, and the trocar 2 at the predetermined meridian position.

FIG. 6 further illustrates that the first portion 62 of the meridian fixing means 60 is in the form of a housing configured to receive and hold, in particular, a shaft 200 of the trocar 2 along the predetermined axis. This may improve the accessibility of the first portion 62 to the required instrument(s). Moreover, as shown in FIG. 6, said first portion 62 and second portion 61 of the meridian fixing means 60 may for example be linked together by a connecting element 63.

FIG. 6 further shows that the second portion 61 of the meridian fixing means 60 comprises two first notches 67a, 67b and two second notches (not shown) provided on opposite sides of the wall, respectively. When the meridian fixing means 60 is mounted in the device 1, as shown in FIG. 1, the two first notches 67a, 67b are disposed on the second dome side and the two second notches (not shown) are disposed on the first dome side. The first notches 67a, 67b and second notches are received in the two grooves 46a, 46b, 56a, 56b of the first dome 40 and the second dome 50 in a movable manner therethrough. The symmetrical configuration of the grooves of the first dome 40 and the second dome 50 improves the mobility of the meridian fixing means 60 along the common meridian slot opening 80.

The configuration of the meridian fixing means 60 according to the embodiment shown in the drawings both facilitates the mobility thereof along the common meridian opening 80 before the fixing means 22, 32 fixes the first dome 40 and the second dome 50 (and thus the trocar 2) at the predetermined angular position and the trocar 2 at the predetermined meridian position, and permits a firm and stable fixing of the trocar 2 at these positions.

The first dome 40 depicted in FIG. 4 further comprises two bumpers 47a; 47b, arranged at the two free ends 40a; 40b of the first dome 40. Correspondingly, FIG. 5 further shows that the second dome 50 of device 1 comprises two notches 57a; 57b arranged at the two free ends 50a; 50b of the second dome 50. The second dome 50 of device 1 possesses two notches 57a; 57b, which are meant to receive the two bumpers 47a; 47b of the first dome 40. This configuration comprising corresponding bumpers 47a; 47b and notches 57a; 57b participates in solidarizing the first and second domes 40; 50 during rotation.

As illustrated for example in FIGS. 7A and 7B, the trocar 2, before being fixed at a predetermined meridian and at a predetermined angular position, may be freely moved to any position along the common meridian slot opening 51, corresponding to any pivoting range within the common meridian slot opening as permitted by the longitudinal dimension of the open annular support, as shown in FIG. 7A, and/or to any angular position on the plane of the open annular support, as shown in FIG. 7B.

Once the trocar 2 is fixed by means of the fixing means 22, 32 at a predetermined meridian and at a predetermined angular position, the trocar does not have to be physically held by a user.

The following description further provides a particular, non-limiting example of a method for fixing a trocar at a predetermined position, the method comprising the steps of:
providing a device 1 for fixing a trocar 2 at a predetermined position according to the first aspect of the present disclosure,
inserting the trocar 2 in the common meridian slot 80 of the first dome 40 and the second dome 50 through the gap 70c of the open annular support 70 along a predetermined axis,
moving the trocar 2 inserted in the common meridian slot 80 through the common meridian slot 80 to a predetermined meridian position,
rotating trocar 2 to a predetermined angular position, and
fixing the trocar 2 at the predetermined meridian position and at the predetermined angular position by means of the fixing means 22, 32.

With reference to the embodiment of the device 1, the method may also comprise the step of inserting the trocar 2 in the first portion 62 of the meridian fixing means 60, by simple clipping. Further, when the open annular support comprises first and second open annular elements, such as in device 1, fixing the trocar comprises engaging the first open annular element 20 with the second open annular element 30. In order to fully block the trocar 2, it may be sufficient to engage the first open annular element 20 with the second open annular element 30 at a predetermined meridian and angular position, by means of the fixing means 22, 32. The fixing means fixes both the first dome 40 and the second dome 50 and thereby the trocar 2 at a desired angular position, and also fixes the trocar in the common meridian slot opening at a predetermined meridian position.

Such engagement between the first and the second open annular elements 20, 30 may imply that the second open annular element 30 comes in contact with the second dome 50, thus tightening the first dome 40 and the second dome 50.

Referring to the example of device depicted in FIG. 1, during operations, the open annular groove 37a may act as a housing for the open annular wall 27 of the first open annular element 20 and the open annular protuberance 37b may act as a tightening agent to induce a pinching of the first dome 40 with the second dome 50, thereby inducing a pinching of the meridian fixing means 60 between the first dome 40 and the second dome 50 and as a consequence, the fixing of the first dome 40, the second dome 50 and thereby the trocar 21 at a predetermined angular position, as well as the fixing of the meridian fixing means 60 and the trocar 2 at a predetermined meridian position.

For example, in the embodiment illustrated by the FIGs. 1-3, the engagement between first and second open annular elements 20, 30 might be carried out by simply applying pressure on the second open annular element 30. When combined with appropriate rotation, such pressure allows the engagement of the protrusions 22 in the notches 32, thereby securing the pinching of the first dome 40 with the second dome 50, thereby inducing a pinching of the meridian fixing means 60 between the first dome 40 and the second dome 50 and as a consequence, the fixing of the first dome 40, the second dome 50 and thereby the trocar 21 at a predetermined angular position, as well as the fixing of the meridian fixing means 60 and the trocar 2 at a predetermined meridian position.

Advantageously as compared to prior art devices, the trocar may be percutaneously inserted into the patient's body prior to being inserted in the device according to one or more embodiments of the present disclosure.

## Claims

1. Device (1) for fixing a trocar (2) at a predetermined position, the device (1) comprising:
an open annular support (70) comprising two free ends (70a, 70b) defining a gap (70c) therebetween,
a first dome (40) rotatably supported by the open annular support (70) and comprising a first meridian slot opening (41) ending in the gap (70c), and
a second dome (50) rotatably supported by the open annular support (70) and comprising a second meridian slot opening (51) ending in the gap (70c),
the first dome (40) and the second dome (50) being juxtaposed to each other so that the second dome (50) is rotatable with the first dome (40) and the first meridian slot opening (41) matches the second meridian slot opening (51) to define a common meridian slot opening (80) for receiving a trocar (2) freely movable therethrough,
the open annular support (70) further comprising a fixing means (22, 32) for fixing the first dome (40) and the second dome (50) at a predetermined angular position and for fixing the trocar (2) received in the common meridian slot opening (80) at a predetermined meridian position.

2. The device according to claim 1, wherein the device (1) further comprises a meridian fixing means (60) for receiving and holding the trocar (2) in the common meridian slot opening (80) along a predetermined axis, the meridian fixing means (60) being movable along the common meridian slot opening (80).

3. The device according to claim 2, wherein the meridian fixing means (60) comprises a first portion (62) and a second portion (61), the first portion (62) being configured to receive and hold the trocar (2) in the common meridian slot opening (80) along the predetermined axis, the second portion (61) being disposed between the first dome (40) and the second dome (50) and being configured to be fixed by the fixing means (22, 32) at the predetermined meridian position.

4. The device according to claim 3, wherein the first portion (62) comprises a housing configured to receive and hold a shaft (200) of the trocar (2) along the predetermined axis.

5. The device according to claim 3 or claim 4, wherein the second portion (61) comprises at least one notch (67a, 67b) and each of the first dome (40) and second dome (50) comprises at least one groove (46a, 46b, 56a, 56b) for receiving the at least one notch (67a, 67b) in a movable manner therethrough, the at least one groove (46a, 46b, 56a, 56b) of each of the first dome (40) and the second dome (50) extending parallel to the respective meridian slot opening (41, 51) of the first dome (40) and the second dome (50).

6. The device according to any one of claims 1-5, wherein the open annular support (70) comprises:
a first open annular element (20) comprising two free ends (20a, 20b) defining a first annular element gap (20c), and
a second open annular element (30) comprising two free ends (30a, 30b) defining a second annular element gap (30c) matching the first annular element gap (20c),
the fixing means (22, 32) comprising:
a first fixing means (22) arranged on the first open annular element (20) and
a second fixing means (32) arranged on the second open annular element (30) and configured to cooperate with the first fixing means (32) to engage the first open annular element (20) with the second open annular element (30).

7. The device according to any one of the preceding claims, wherein the first fixing means (22) and the second fixing means (32) comprise a notch and a protrusion, respectively, or viceversa.

8. The device according to any one of claims 1-6, wherein the first fixing means (22) and the second fixing means (32) comprise a tapping and a threading, respectively, or viceversa.

9. The device according to any one of the preceding claims, wherein the first dome (40) is provided with a first open annular edge (44) rotatably supported by the open annular support (70) and comprising two free ends (40a, 40b) defining a first annular edge gap (40c) therebetween and the second dome (50) is provided with a second open annular edge (54) rotatably supported by the open annular support (70) and comprising two free ends (50a, 50b) defining a second annular edge gap (50c) therebetween, the first annular edge gap (40c) matching the second annular edge gap (50c).

10. The device according to claim 9, wherein the first dome (40) further comprises at least one bumper (47a, 47b) arranged along the common meridian slot (80) at the proximity of at least one of the two free ends (40a, 40b) of the first dome (40).

11. The device according to claim 10, wherein the second dome (50) comprises at least one notch (57a, 57b) arranged along the common meridian slot (80) at the proximity of at least one of the free ends (50a, 50b) of the second dome (50), the at least one notch (57a, 57b) receiving the at least one bumper (47a, 47b).

12. Method for fixing a trocar (2) at a predetermined position, the method comprising the steps of:
providing a device (1) for fixing a trocar (2) at a predetermined position according to any one of the preceding claims,
inserting the trocar (2) in the common meridian slot (80) of the first dome (40) and the second dome (50) through the gap (70c) of the open annular support (70) along a predetermined axis,
moving the trocar (2) inserted in the common meridian slot (80) through the common meridian slot (80) to a predetermined meridian position,
rotating trocar (2) to a predetermined angular position,
fixing the trocar (2) at the predetermined meridian position and at the predetermined angular position by means of the fixing means (22, 32).

13. The method of claim 12 when the device (1) is according to any one of claims 3-11, further comprising inserting the trocar (2) in the first portion (62) of the meridian fixing means (60).

14. The method of claim 12 when the device (1) is according to any one of claims 6-11, wherein fixing the trocar (2) comprises engaging the first open annular element (20) with the second open annular element (30).
